Europäisches Patentamt

(19) European Patent Office

Office européen des brevets

(11) Publication number: **0 082 596**
**A1**

## (12) EUROPEAN PATENT APPLICATION

(21) Application number: **82306042.1**

(22) Date of filing: **12.11.82**

(51) Int. Cl.³: **A 61 M 13/00**
**A 61 M 5/00, A 61 M 25/02**

(30) Priority: **27.11.81 US 325169**

(43) Date of publication of application:
**29.06.83 Bulletin 83/26**

(84) Designated Contracting States:
**DE FR GB IT**

(71) Applicant: **BAXTER TRAVENOL LABORATORIES, INC.**
**One Baxter Parkway**
**Deerfield, IL 60015(US)**

(72) Inventor: **Bellamy, David, Jr.**
**92 Robsart Road**
**Kenilworth Illinois 60043(US)**

(74) Representative: **MacGregor, Gordon et al,**
**ERIC POTTER & CLARKSON 14 Oxford Street**
**Nottingham, NG1 5BP(GB)**

(54) Aseptic isolation of a skin site.

(57) An infection-prone living body site may be rendered substantially sterile by sealing a bacteria-resistant enclosure (10) about the body site and passing gas into the enclosure for bacteriocidal or bacteriostatic effect. Apertures (24, 26) having bacteria filters (28) are used for gas entry and exit.

FIG. 2

EP 0 082 596 A1

## ASEPTIC ISOLATION OF A SKIN SITE

Technical Field

This invention relates to a method and apparatus for rendering an infection-prone living body site substantially sterile.

In some varieties of peritoneal dialysis, for example, the technique known as continuous ambulatory peritoneal dialysis, an indwelling catheter is placed in the peritoneal cavity, communicating through the skin and the stomach wall on a permanent or semipermanent basis. In other medical situations, indwelling intravenous catheters and the like are used.

As a disadvantage of indwelling catheters, they can be a significant cause of infection, due to the migration of microorganisms into the incision in which the catheter is placed.

This situation becomes a critical problem in the maintenance of tumor patients, for example, who are undergoing chemotherapy so that their immunological system is disabled. In this case, the possibilities of infection due to bacteria migration along the wall of a catheter into the catheter entry site can be critical and life threatening.

Accordingly, there is a need for providing substantial sterility to an infection-prone site such as a catheter entry incision site in an immunologically deficient person. By this invention, a bacteria-impermeable enclosure can be provided in which sterilizing gas can be applied at the living body site for continuous or repeated antibacterial effect. Thus the incision site, or any other infection-prone living body site, can be protected.

Additionally a means is provided to make the environment immediately surrounding the entry incision site less supportable of any bacterial growth from residual bacteria which may have survived any germicidal action, by reduction of the moisture content of said environment.

The words "sterile" and similar words as used herein are intended to include not just the concept of a total absence of the microorganisms, but they are also intended to include the concept of substantial sterility, in which the number of microorganisms is reduced to such a low populaton that the likelihood of infection or contamination is substantially reduced or eliminated.

The method and apparatus of this invention relates to the passage of sterilizing gas into and optionally out of an enclosure, for sterilizing purposes and/or a dehumidifying gas into and out of an enclosure for dehumidifying purposes.

Description of Prior Art

It is common practice in the manufacture of various sterile hospital dressings, procedure trays, and the like to package the item in a wrapping which includes at least in part a porous paper member. The sealed package is then exposed to ethylene oxide gas, which diffuses through the porous paper (which is of a pore size to block bacteria) so that the ethylene oxide gas sterilizes the contents after they have been wrapped and sealed in their outer wrapping. The ethylene oxide then diffuses out again from the package. Thus the packaged item is capable of being handled in a normal environment, while the interior remains sterile.

The invention of this application relates to the maintenance of substantially sterile conditions at an infection-prone living body site, for example a portion of the skin of a patient, in a manner quite different from the sterilization of packaged medical items.

The invention of this application may find particular use for maintaining substantially sterile conditions at a catheter entry site into the body where the catheter resides for long periods of time, and the possibility of infection is great unless countermeasures are taken.

Typical countermeasures which are taken in current technology include the application of antiseptic ointment on the catheter entry site, followed by the covering of the antiseptic ointment by bandages and the like. However, a disadvantage of this is that such a dressing must be frequently changed. Under the antiseptic ointment, a wet bed forms on the skin which can serve as a site for the proliferation of bacteria when the antiseptic of the ointment becomes exhausted, or in the event the bacteria become resistant to the antiseptic.

However, on the changing of each dressing, there is a possibility that infection can be transmitted to the infection prone site, such as an entry site for a catheter. Since the wound site is perpetually moist, being covered over by the ointment except during the brief periods of changing of the ointment and dressing, the possibility for bacterial proliferation still exists despite attempts to prevent it.

Disclosure of Invention

In this invention a method is provided for rendering substantially sterile an infection-prone living body site, such as the skin surrounding an incision for a catheter. A bacteria-resistant enclosure is sealed about the body site, and sterilizing gas is passed into the enclosure to render the body site substantially sterile.

Typically, the enclosure comprises a sealing wall defining a peripheral flange about its entire periphery for sealing about the body site, to enclose it in a bacteria-impermeable sealed zone. The peripheral flange may carry adhesive for forming an adhering seal with the skin, or, alternatively, the flange may be taped to the skin. Also, the flange may be omitted and the enclosure strapped or otherwise adhered to the skin.

One and preferably two or more aperture means, each having bacteria filter means, may be provided in the enclosure wall to respectively serve as a gas inlet and a gas outlet. Means, connectable to the aperture for passing antiseptic gas into the zone sealed by the enclosure wall, are provided for communication with one of the aperture means so that the antiseptic gas may pass inwardly through the bacteria filter to fill the enclosure, thus having bacteriocidal effect on the body site enclosed by the enclosure.

The second aperture defined in the enclosure may serve as an exit for antiseptic gas if desired.

It may also be advantageous to blow a nonreactive drying gas such as nitrogen gas or air through the enclosure, typically through one of the apertures and out the other, to substantially dry the body site which, of course, provides further limitation to the possibility of the spread of infection. This blowing step may be continuous if desired, or intermittent as the case may be. The nonreactive drying gas may also serve as a vehicle to propel and distribute the antiseptic gas into the enclosure, and then to blow the antiseptic gas out of the enclosure after the desired antibacterial effect has been achieved, to avoid undue irritation of the skin. Also, antiseptic gas may be omitted, if desired, with only the nonreactive gas being used to dry the site for bacteriostatic effect.

Types of antiseptic gases which may be used herein include essentially any antiseptic gas suitable for the purpose. Halogen gases may be used, particularly chlorine, but also bromine and iodine, for example. Chlorine oxides may be used, or HClO vapor. Also, nascent oxygen, particularly ozone, but also oxygen as available from peroxide forms, oxygen ions having sterilizing effect, or atomic oxygen may be used, where desired.

The nonreactive drying gases which may be used as described above, to be blown through the enclosure, include air, nitrogen, carbon dioxide, or oxygen gas.

Oxygen gas, of course, may be deemed to be reactive toward the destruction of bacteria, depending upon the type of bacteria, so that in certain circumstances it may constitute a sterilizing gas as well as a nonreactive gas as used in this invention.

Preferably, at least a portion of the enclosure is transparent so that the body site may be viewed from the exterior. The sterilizing gas such as chlorine or ozone may be inserted from time to time through one of the apertures carrying the bacteria filter means, allowed to reside in the enclosure for a predetermined period of time for antibacterial activity, and then blown out of the enclosure by means of a nonreactive gas. The dry, nonreactive gas such as compressed air may be blown through the enclosure on a continuous or intermittent base to a degree sufficient to substantially dry the body site. When the enclosure has at least a transparent section, the appearance of condensation on the inner wall of the transparent section can be used as an indication that more gas blowing is required to dry out the body site for suppression of bacterial growth.

The enclosure may also define a catheter entry port proportioned to receive a catheter passing from the exterior into the sealed zone while defining a bacteria-blocking seal between the sealing wall and the catheter at the entry port. Thus the catheter entry site into the tissue may be sealingly enclosed in a bacteria-blocking manner, and intermittently subjected to sterilizing gas and a current of sterile inert gas, as may be desired.

Brief Description of the Drawings

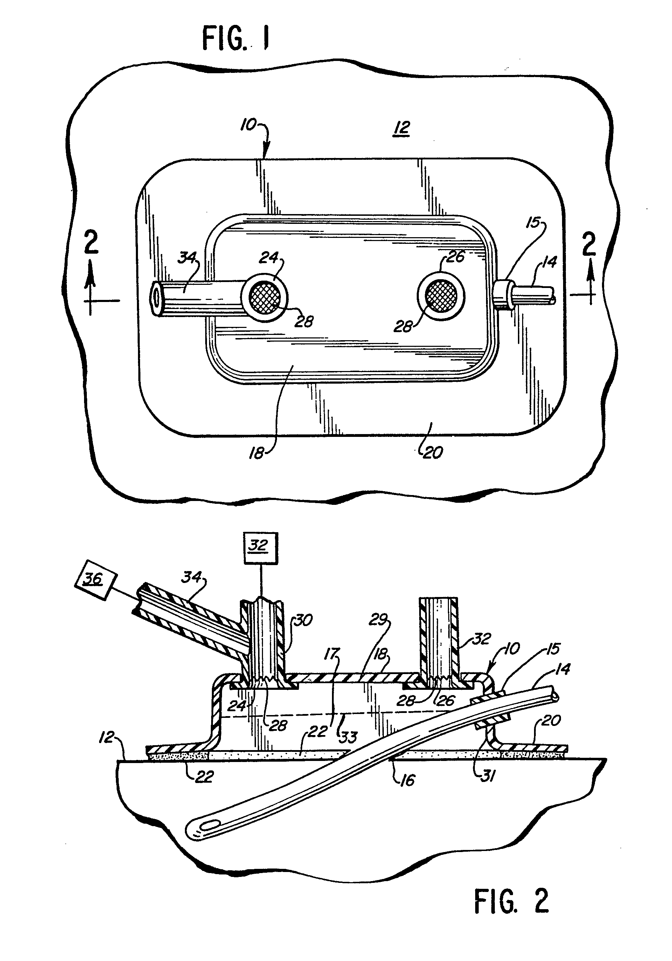

Figure 1 is a plan view of the bacteria-blocking enclosure of this invention.

Figure 2 is a sectional view taken along line 2-2 of Figure 1, with some portions shown schematically.

### Description of Specific Embodiment

Referring to the drawings, bacteria-blocking enclosure 10 is shown sealed in place upon a portion of the skin 12 of a patient. For example, the patient may be undergoing chemotherapy, resulting in a severely impaired immunological function, so that he is extremely prone to infection. For therapeutic purposes, it may be necessary for the patient to carry a indwelling catheter 14 extending through sealing port 15 in enclosure 10, and also the skin 12 through an incision site 16. Since this incision site serves as a possible site for an infection of such a patient of life-threatening proportions, it is surrounded by enclosure 10.

Enclosure 10 may comprise a piece of polyvinyl chloride plastic or other plastic, for example comprising a sealing wall 18 and defining a peripheral flange 20 about its entire periphery. Flange 20 may carry adhesive 22 which sticks to the skin 12 to enclose the body site including incision 16 in a bacteria-impermeable sealed zone 17.

A pair of aperture means or ports 24, 26 are shown to be defined through sealing wall 18, each aperture carrying a bacteria-blocking filter 28, which may be a conventional hydrophobic 0.22 micron filter of a type commercially used in biomedical devices, with its hydrophobic properties preventing the migration of aqueous liquids through the filter.

If desired, enclosure 10 may comprise upper and lower separable, sealable pieces 29, 31 so that it may be assembled around catheter 14 for application after emplacement of the catheter without withdrawal of the catheter from its incision site. In that case the upper part of sealing port 15 is part of piece 29 and the lower part of port 15 is part of piece 31. Pieces 29, 31 are sealed together when installed along annular, peripheral seal line 33.

Aperture 24 may communicate with branched tubing, one branch 30 being capable of communication in conventional manner to a source 32 of compressed, dry, inert gas, for example a tank of compressed air or nitrogen. Accordingly, dry air or nitrogen can sweep through tubing 30 and filter 28 to pass through the enclosure 10, and to exit through aperture 26 which carries filter 28 and optional tubing 32.

Branch line 34 may be provided as well, or alternatively branch line 34 may communicate directly by a third aperture through the wall of enclosure 10, being preferably equipped in that instance with a third bacteria-blocking filter. Branch line 34 leads to a source of antiseptic gas, for example chlorine or ozone. The chlorine or ozone gas may be pressurized if desired by any means so that it easily flows into enclosure 10. Alternatively, the flow of nonreactive gas 32 through tubing 30 can generate an aspiration in branch line 34 that will draw the sterilizing gas from its source 36 into the interior of enclosure 10.

After an adequate supply of sterilizing gas has been inserted into enclosure 10, it may be desired to turn off the flow of gases to allow the sterilizing gas to have time to act. Thereafter, if desired, resumption of the flow of nonreactive gas from source 32 may continue to sweep any remaining sterilizing gas out of enclosure 10 through exhaust aperture 26. Thereafter, a continuous flow of nonreactive gas may be swept through the system if desired, or intermittent bursts of gas may be utilized to reduce the moisture content of the zone 17 within enclosure 10.

The antiseptic gas may be provided intermittently or continuously as may be desired, with or without added nonreactive gas. Also the nonreactive gas may be used without antiseptic gas to dry the site.

Tubes 30, 32 and 34 may optionally be valved or capped off between use to minimize challenge to the bacterial filters.

CLAIMS

1.    Apparatus for use in protecting an infection-prone body
site from infection, characterised by a bacteria-resistant
enclosure (10) having a sealing wall (18) for sealing about
the body site to enclose the latter in a bacteria-resistant
sealed zone, aperture means (24, 26) in the wall permitting
passing of sterilising gas into the enclosure, and bacteria
filter means (28) blocking the aperture means.

2.    Apparatus according to claim 1 in which the enclosure
(10) has a catheter entry port (15) proportioned to receive a
catheter (14) passing from the exterior into the sealed zone
and defining a bacteria seal between the sealing wall (18)
and the catheter.

3.    Apparatus according to Claim 1 or 2 which includes a
pair of  aperture means (24, 26), each having bacteria filter
means (28), to serve respectively as a gas inlet and a gas
outlet.

4.    Apparatus according to claim 3 together with means (36)
for providing antiseptic gas to one of the aperture means.

5.    Apparatus according to Claim 4 together with means (32)
for providing nonreactive gas to the aperture means serving
as an inlet

6.    Apparatus according to Claim 3, 4 or 5 including a
branched inlet to the aperture means (24) having a branch
(30) for nonreactive gas and a branch (34) for sterilising
gas.

7.    Apparatus according  to any preceding claim, having a peripheral flange (20) about its entire periphery and carrying adhesive for sealing about the said body site.

8. Apparatus according to any preceding claim in which at least a portion of the enclosure is transparent.

9. Apparatus according to any preceding claim wherein the enclosure (10) is separable into two pieces (29, 31) assemblable about a catheter (14) emplaced in the body site without removing the catheter from the body site.

10. Apparatus according to any preceding claim in which the or each filter means (28) is a bacteria-blocking hydrophobic filter having a pore size of about 0.2 micron.

11. A method of rendering substantially sterile an infection-prone living body site, comprising sealing a bacterial-resistant enclosure (10) about the body site and passing sterilizing gas into the enclosure to render the body site substantially sterile.

0082596

1/1

FIG. 1

FIG. 2

## PARTIAL EUROPEAN SEARCH REPORT
which under Rule 45 of the European Patent Convention shall be considered, for the purposes of subsequent proceedings, as the European search report

European Patent Office

Application number

EP 82306042.1

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| Y | US - A - 3 874 387 (P. BARBIERI)<br>* Fig. 2; column 2, lines 4-32 * | 1,3,7,10 | A 61 M 13/00<br>A 61 M 5/00<br>A 61 M 25/02 |
| Y | US - A - 4 271 973 (D.A. QUAGLIARO)<br>* Fig. 2; column 2, lines 48-56 * | 1,3,7,10 | |
| A | GB - A - 1 150 294 (GEN.EL.COMP.)<br>* Fig. 2; page 2, line 39 - page 3, line 2 * | 1,3,4,7 | TECHNICAL FIELDS SEARCHED (Int. Cl.³) |
| A | US - A - 3 194 235 (E.A. COOKE)<br>* Totality * | 1,2 | A 61 F 13/00<br>A 61 M 1/00<br>A 61 M 5/00<br>A 61 M 13/00 |
| A | GB - A - 2 046 095 (OUTCASTLE LTD.)<br>* Totality * | 1,2,7,8 | A 61 M 25/00<br>A 61 M 27/00 |
| A | GB - A - 435 358 (W. STAHEL)<br>* Page 1, lines 9-11 * | | A 61 M 35/00<br>A 61 M 37/00<br>B 65 D 51/00 |

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-10
Claims searched incompletely: —
Claims not searched: 11
Reason for the limitation of the search:

According to Article 52(4)

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family,
corresponding document

| Place of search | Date of completion of the search | Examiner |
|---|---|---|
| VIENNA | 29-03-1983 | LUDWIG |

EPO Form 1505.1   06.78